# EUROPEAN PATENT SPECIFICATION

(11) **EP 3 751 282 B1**
(45) Date of publication and mention of the grant of the patent: **15.12.2021**
(21) Application number: 19180404.6
(22) Date of filing: 14.06.2019
(51) Int. Cl.: G01N 33/36, G01N 27/22

(54) **CAPACITIVE YARN SENSOR DEVICE WITH OFFSET COMPENSATION**
KAPAZITIVER GARNSENSOR MIT OFFSETKOMPENSATION
CAPTEUR DE FIL CAPACITIF AVEC COMPENSATION DE DÉCALAGE

(43) Date of publication of application: 16.12.2020
(73) Proprietor: Gebrüder Loepfe AG, 8623 Wetzikon (CH)
(72) Inventor: Knechtle, Stefan, 8623 Wetzikon (CH); Schnaubelt, Matthias, 8315 Lindau (CH)
(74) Representative: Sutter, Kurt

(56) References cited:
- EP-A1- 2 108 949
- US-A- 4 208 625

## Description

### Technical Field

The invention relates to a capacitive sensor device for measuring at least one parameter of an elongate textile body, in particular at least one parameter of a yarn.

In particular, such a sensor device can measure a parameter describing the volume of the body in the measurement region or variations thereof.

### Background Art

WO 2010/043065 and EP 2352018 describe a capacitive sensor device according to the preamble of the independent claim. In particular, the sensor device comprises a measurement volume for receiving the body and a capacitive measurement assembly comprising a measurement capacitor and a reference capacitor in series. The measurement capacitor has electrodes arranged on opposite sides of the measurement volume. The signal of an AC voltage generator is applied over the two capacitors. The potential between the measurement capacitor and the reference capacitor forms the output of the measurement assembly and carries an output signal. The input of a signal amplifier is connected to the output of the measurement assembly.

The device further has an offset compensation circuit with a control unit and an offset compensator. The offset compensator is adapted to compensate the asymmetry between the measurement capacitor and the reference capacitor. The control unit is adapted to control the offset compensator in response to the amplitude of said output signal.

The offset compensator is arranged in the signal path between the AC voltage generator and the reference and/or measurement capacitor, i.e. it is arranged before the measurement assembly.

### Disclosure of the Invention

The problem to be solved by the present invention is therefore to provide an alternative solution for compensating the asymmetry of a sensor device of the type mentioned above.

This problem is solved by the sensor device of claim 1. Accordingly, the sensor device comprises:
- A measurement volume. This volume is adapted to receive the textile body during the measurement.
- An AC voltage generator generating an AC measurement voltage at a master frequency.
- A capacitive measurement assembly: The measurement assembly comprises a measurement capacitor and a reference capacitor arranged in series. The measurement capacitor comprises electrodes arranged on opposite sides of the measurement volume, i.e. its capacitance is a function of the parameter to be measured. The measurement assembly has an output for generating an output signal. The output is located between the measurement capacitor and the reference capacitor.
- A signal amplifier for amplifying the output signal. The input of the signal amplifier may be directly coupled to the output of the measurement assembly, or there may be further components, e.g. for filtering, between the input of the signal amplifier and the output of the measurement assembly.
- An offset compensation circuit comprising a control unit and an offset compensator: The offset compensator is adapted to compensate the asymmetry between the measurement capacitor and the reference capacitor. The control unit is adapted to control the offset compensator in response to the amplitude of the output signal of the measurement assembly.

According to the invention, the offset compensation unit comprises at least the following components:
- A compensation waveform generator generating an AC compensation signal: The compensation signal is in synchronicity with the measurement voltage of the voltage generator, i.e. it has the same period, but it may have a non-zero phase-offset in respect to the measurement voltage. This compensation waveform generator is connected to a correction point (P1). The correction point is a point on the signal path from the output of the measurement assembly to the input of the signal processor. Specifically, the correction point may located be directly at the output of the measurement assembly, directly at the input of the signal amplifier, or at any point on the signal path between these two locations.

This allows to efficiently and in a simple manner compensate an asymmetry, i.e. to offset the signal from the measurement assembly in such a way that it e.g. falls into the working range of the signal amplifier or any components after the signal amplifier.

In an advantageous embodiment, a coupling capacitor is arranged in the signal path between the compensation waveform generator and the correction point, thus capacitively coupling the compensation waveform generator to the correction point.

Advantageously, the control unit is adapted to change the amplitude of the compensation signal for compensating the asymmetry.

The invention also relates to a method for offset compensating the sensor device. This method includes at least the following steps:
- Measuring a monitor value indicative of an amplitude of the output signal of said signal amplifier.
- Reducing this monitor value by changing the compensation signal.

Advantageously, in the above first step, the measurement volume is operated with a constant load, in particular without the elongate body present in the measurement volume. Alternatively, the monitor value is averaged over a time span much larger than typical signal fluctuations caused by the varying properties of the elongate body.

Advantageously, in the above second step, the monitor value is zeroed, i.e. the amplitude of the output signal of the signal amplifier at the frequency of the AC voltage generator is adjusted to zero.

All AC signals mentioned herein are, unless otherwise noted, advantageously measured at the master frequency, i.e. the (fundamental) frequency of the measurement voltage.

Other advantageous embodiments are listed in the dependent claims as well as in the description below.

### Brief Description of the Drawings

The invention will be better understood and objects other than those set forth above will become apparent from the following detailed description thereof. Such description refers to the annexed drawings, wherein:
Fig. 1 shows a block diagram of the sensor device including a sectional view of a measuring head,
Fig. 2 is a circuit diagram of an embodiment of the sensor device,
Fig. 3 is a timing diagram showing the measurement signal i1 as well as a compensation signal i2, and
Fig. 4 illustrates an alternative to the scheme of Fig. 3.

### Modes for Carrying Out the Invention

### Sensor device

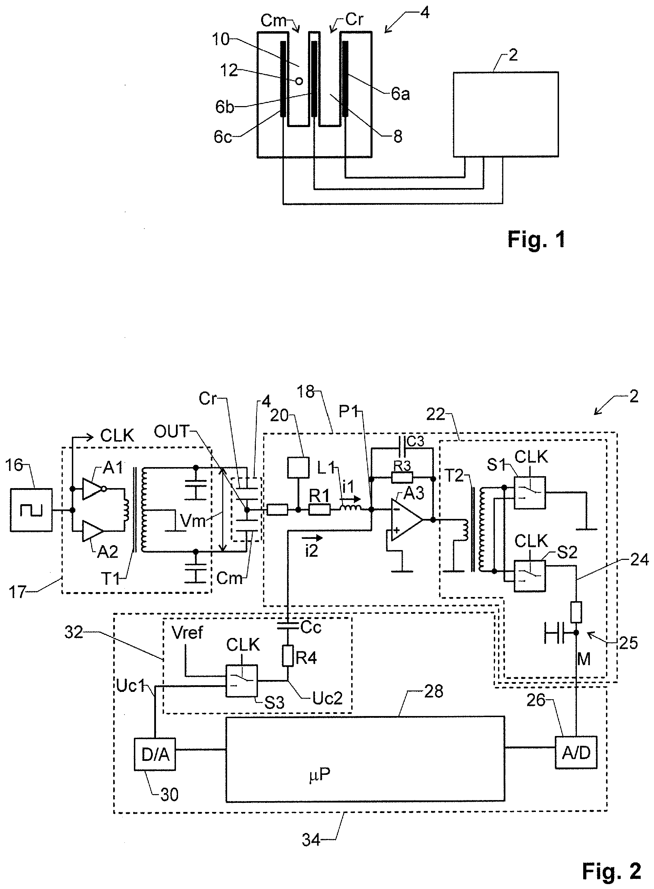

The sensor device as shown in Fig. 1 comprises control circuitry 2 connected to a measuring head 4.

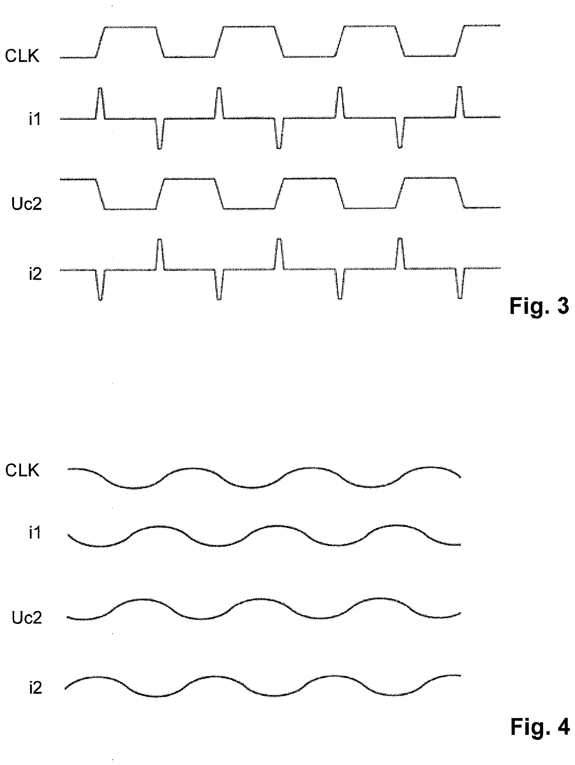

Measuring head 4 is e.g. a device as shown in Fig. 4 of US4843879. It comprises at least three electrodes 6a, 6b, 6c forming a reference capacitor Cr and a measurement capacitor Cm in a series configuration.

The electrodes 6a, 6b of reference capacitor Cr are arranged on opposite sides of a reference volume 8 while the electrodes 6b, 6c of measurement capacitor Cm are arranged on opposite sides of a measurement volume 10.

Measurement volume 10 is open at least on two opposite sides for receiving an elongate textile body 12, such as a yarn. Advantageously, measurement volume 10 forms a slit that is closed on three sides and open on three sides.

Reference volume 8 may be closed or it may also form a slit exposed to the same environment as measurement volume 10.

During the measurement, textile body 12 is running along its longitudinal axis (which extends perpendicular to the drawing of measurement assembly 4 of Fig. 1). The capacitance of measurement capacitor Cm varies as a function of the volume and the dielectric properties of body 12 within measurement volume 10.

As e.g. described in US4843879, this allows to determine a parameter of body 12, such as its average cross section or volume in measurement volume 10.

### Circuit Design

Fig. 2 shows an embodiment of a circuit as used in the sensor device.

The circuit comprises a digital or analog oscillator 16 generating a clock signal CLK. Advantageously, the fundamental frequency (in the following called the "master frequency f0") of this signal is in the MHz range, in particular but not limiting between 1 ― 100 MHz, in particular 1 ― 30 MHz.

Clock signal CLK is used by a voltage generator 17 for generating an alternating measurement voltage Vm at master frequency f0 across the series arrangement of measurement capacitor Cm and reference capacitor Cr.

In the shown embodiment, this is achieved by feeding clock signal CLK to a complementary amplifier A1, A2 and a first transformer T1. Other suitable circuits are known to the skilled person.

The point between the two capacitors Cm,. Cr forms the output OUT of measuring head 4 and generates its AC output signal.

If the capacitors Cm and Cr are balanced, i.e. their capacitances are equal, the output signal is equal zero, i.e. the current flowing through the output OUT is zero.

The signal from output OUT is fed to a signal processor 18.

At its input, signal processor 18 e.g. comprises a voltage limiter 20 protecting the downstream circuitry from excessive voltages.

Next, the output signal may pass first filter elements R1 and L1 and a signal amplifier A3 with feedback elements R3, C3.

The output of signal amplifier A3 is fed to a lock-in amplifier 22, which selectively amplifies the signal at master frequency f0 as it is known to the skilled person.

Various designs for lock-in amplifiers are known to the skilled person. The one shown here uses a voltage-amplifying transformer T2 and two electronically controlled switches S1, S2 controlled by clock signal CLK.

The output signal 24 is passed through a low-pass filter 25 for generating a monitor value M and then to an analog-digital converter 26 and to a control unit 28.

Control unit 28 may be a microprocessor with a memory circuit that stores data and program instructions. It is programmed to carry out the offset compensation steps for the sensor device.

Control unit 28 is connected to a digital-analog-converter 30 for generating a first control voltage Uc1, which is fed to an offset compensator 32.

Control unit 28 and offset compensator 32 form the core parts of an offset compensation circuit 34 that is used for compensating any excessive asymmetry in measurement assembly 4 in order to keep signal amplifier A3 and lock-in amplifier 22 in their permissible operating range while still allowing for a high signal amplification.

In the embodiment of Fig. 2, offset compensator 32 comprises an electronically controlled switch S3 controlled by clock signal CLK. Switch S3 forms a compensation waveform generator. At its output, it generates a second control voltage Uc2, whose value oscillates between a fixed reference voltage Vref and first control voltage Uc1.

Second control voltage Uc2 is fed through a resistor R4 and a coupling capacitor Cc, which are connected in series, to a correction point P1.

Correction point P1 is located in the signal path from output OUT of measuring head 4 to the input of signal amplifier A3.

Coupling capacitor Cc converts voltage Uc2 to a current i2. Current i2 has advantageously a similar shape as the current i1 of an asymmetrical capacitive measurement assembly 4. With proper setting of the D/A, current i2 has the same amplitude with inverse sign (i1=-i2). Resistor R4 may be added to delay current i2 slightly to achieve currents i1 and i2 having same phase.

The current i2 coming from compensation capacitor Cc is in the following called the "compensation signal". The signal at the output of signal amplifier A3 is proportional to the sum of this compensation signal i2 and the current i1 on the signal path from output OUT of measuring head 4 to the input of signal amplifier A3.

### Offset Compensation

Fig. 3 illustrates clock signal CLK, signal current i1, second control voltage Uc2, and compensation signal i2 as a function of time.

The values of coupling capacitor Cc and resistor R4 are advantageously chosen such that current i1 and compensation signal i2 have, at master frequency f0, substantially a phase shift of 0° or 180° (e.g. within +/-45°).

Hence, the time constant τ = Cc·R4 of the series arrangement of capacitor Cc and resistor R4 is advantageously between 0 and 0.4/f0, i.e. depending on the desired phase shift, Cc·R4/f0 should be between 0 and 0.4.

Signal amplifier A3 amplifies the sum of the signals i1 and i2. Hence, an asymmetry in measuring head 4, which leads to a non-zero value of the amplitude of signal current i1 at master frequency f0, can be compensated by suitably increasing or decreasing the compensation signal i2.

As mentioned, offset compensation circuit 34 is designed to keep the effect of an asymmetry of the capacitances Cm and Cr sufficiently small so that signal amplifier A3 and any subsequent components, such as lock-in amplifier 22, remain within their operating ranges

For this purpose, compensation circuit 34 measures the voltage value at output M of processing unit 18, which is proportional to the amplitude of the output voltage of signal amplifier A3 at master frequency f0.

If the monitor value M is too large (indicating that the output amplitude of signal amplifier A3 or lock-in amplifier 22 are close to their permissible ranges), control unit 28 changes first control voltage Uc1, which changes the amplitude of second control voltage Uc2 and therefore the amplitude of compensation signal i2. Depending on the mutual signs of signal current i1 and compensation signal i2, this can lead to an increase or a decrease of monitor value M.

If control unit 28 detects that a given change of first control voltage Uc1 leads to an increase of the monitor value M, it changes Uc1 into the opposite direction.

Hence, by suitably changing control voltage Uc1, control unit 28 is able to reduce monitor value M.

Advantageously, monitor value M is zeroed, i.e. it is changed to a value indicative that the amplitude of the output voltage of signal amplifier A3 at the master frequency f0 is zero.

### Notes

In the above embodiment, coupling capacitor Cc is arranged closer to correction point P1 than resistor R4. As it is clear to the skilled person, the order of these components may be swapped, though.

Further, resistor R4 may be omitted if second control voltage Uc2 is suitable phase-shifted by other means or if there is no significant phase shift between the currents i1 and i2.

In the embodiment of Figs. 2 and 3, second control voltage Uc2 is amplitude-modulated, with an amplitude given by the difference of Vref and first control voltage Uc1.

Generally, the compensation signal, i.e. current i2, is advantageously inverse to the measurement signal i1. This can be achieved by either operating compensation circuit 34 with a suitably shifted clock signal and/or by inducing a phase shift in the compensation signal.

Note that the pulse generator may also generate a signal with a fixed voltage, which is then varied by varying R4 and/or Cc, e.g. by using an electronically variable resistor and/or an electronically variable capacitor.

It must be noted that compensation circuit 34 can e.g. also be designed to generate sine currents or other waveforms.

Fig. 4 shows an embodiment where it assumed that the clock signal is a sine signal and the currents i1 and i2 are sine signals, too.

While there are shown and described presently preferred embodiments of the invention, it is to be distinctly understood that the invention is not limited thereto but may be otherwise variously embodied and practiced within the scope of the following claims.

## Claims

1. A capacitive sensor device for measuring at least one parameter of an elongate textile body, in particular of a yarn, said sensor device comprising
a measurement volume (10) for receiving the textile body,
an AC voltage generator (17) adapted to generate an AC measurement voltage (Vm) at a master frequency (f0),
a capacitive measurement assembly (4) comprising a measurement capacitor (Cm) and a reference capacitor (Cr) arranged in series, wherein said measurement capacitor (Cm) comprises electrodes (6b, 6c) arranged on opposite sides of said measurement volume (10), and wherein said measurement assembly (4) has an output (OUT) adapted to generate an output signal, wherein said output is located between the measurement capacitor (Cm) and the reference capacitor (Cr),
a signal amplifier (A3) for amplifying said output signal,
an offset compensation circuit (34) comprising a control unit (28) and an offset compensator (32), wherein said offset compensator (32) is adapted to compensate an asymmetry between said measurement capacitor (Cm) and said reference capacitor (Cr) and wherein said control unit (28) is adapted to control said offset compensator (32) in response to an amplitude of said output signal,
**characterized in that** said offset compensator (32) comprises
a compensation waveform generator (S3) adapted to generate an AC compensation signal (i2) in synchronicity with said measurement voltage (Vm),
wherein said compensation waveform generator (S3) is connected to a correction point (P1) located along a signal path from the output (OUT) of said measurement assembly (4) to an input of said signal amplifier (A3).

2. The sensor device of claim 1 further comprising a coupling capacitor (Cc) between the compensation waveform generator (S3) and the correction point (P1).

3. The sensor device of any of claim 2 further comprising a resistor (R4) in series with said coupling capacitor (Cc).

4. The sensor device of claim 2 wherein a capacitance Cc of said coupling capacitor (Cc), a resistance R4 of said resistor (R4), and said master frequency f0 fulfil 0 < Cc·R4/f0 < 0.4.

5. The sensor device of any of the preceding claims wherein said control unit (28) is adapted to change an amplitude of said compensation signal (i2) for compensating said asymmetry.

6. A method for offset compensating the sensor device of any of the preceding claims comprising the steps of
measuring a monitor value (M) indicative of an amplitude of an output signal of said signal amplifier (A3) and
reducing said monitor value (M) by changing said compensation signal (i2).

7. The method of claim 6 wherein said monitor value (M) is zeroed by changing said compensation signal (i2).

## Patentansprüche

1. Eine kapazitive Sensorvorrichtung zur Messung mindestens eines Parameters eines langgestreckten Textilkörpers, insbesondere eines Fadens, wobei die Sensorvorrichtung Folgendes umfasst:
ein Messvolumen (10) zur Aufnahme des Textilkörpers,
einen Wechselspannungsgenerator (17), der zur Erzeugung einer Messwechselspannung (Vm) mit einer Leitfrequenz (f0) ausgestaltet ist,
eine kapazitive Messanordnung (4), die einen Messkondensator (Cm) und einen Referenzkondensator (Cr) umfasst, die in Reihe angeordnet sind, wobei der Messkondensator (Cm) Elektroden (6b, 6c) umfasst, die auf gegenüberliegenden Seiten des Messvolumens (8) angeordnet sind, und wobei die Messanordnung (4) einen Ausgang (OUT) aufweist, der zur Erzeugung eines Ausgangssignals ausgestaltet ist, wobei der Ausgang zwischen dem Messkondensator (Cm) und dem Referenzkondensator (Cr) angeordnet ist,
einen Signalverstärker (A3) zur Verstärkung des Ausgangssignals,
eine Offset-Kompensationsschaltung (34), die eine Steuereinheit (28) und einen Offset-Kompensator (32) umfasst, wobei der Offset-Kompensator (32) zur Kompensation einer Asymmetrie zwischen dem Messkondensator (Cm) und dem Referenzkondensator (Cr) ausgestaltet ist, und wobei die Steuereinheit (28) zur Steuerung des Offset-Kompensators (32) in Reaktion auf eine Amplitude des Ausgangssignals ausgestaltet ist,
**dadurch gekennzeichnet, dass** der Offset-Kompensator (32) Folgendes umfasst
einen Kompensationswellenformgenerator (S3), der zur Erzeugung eines Wechselspannungskompensationssignals (i2) synchron mit der Messspannung (Vm) ausgestaltet ist,
wobei der Kompensationswellenformgenerator (S3) mit einem Korrekturpunkt (P1) verbunden ist, der sich entlang eines Signalwegs vom Ausgang (OUT) der Messanordnung (4) zu einem Eingang des Signalverstärkers (A3) befindet.

2. Die Sensorvorrichtung nach Anspruch 1, weiter umfassend einen Kopplungskondensator (Cc) zwischen dem Kompensationswellenformgenerator (S3) und dem Korrekturpunkt (P1).

3. Die Sensorvorrichtung nach einem der Ansprüche 2, weiter umfassend einen Widerstand (R4) in Reihe mit dem Kopplungskondensator (Cc).

4. Die Sensorvorrichtung nach Anspruch 2, wobei eine Kapazität Cc des Kopplungskondensators (Cc), ein Widerstand R4 des Widerstands (R4) und die Leitfrequenz f0 0 < Cc·R4/f0 < 0.4 erfüllen.

5. Die Sensorvorrichtung nach einem der vorangehenden Ansprüche, wobei die Steuereinheit (28) zur Änderung einer Amplitude des Kompensationssignals (i2) ausgestaltet ist, um die Asymmetrie zu kompensieren.

6. Ein Verfahren zur Offsetkompensation der Sensorvorrichtung nach einem der vorangehenden Ansprüche, umfassend die folgenden Schritte
Messen eines Überwachungswertes (M), der eine Amplitude eines Ausgangssignals des Signalverstärkers (A3) anzeigt, und
Reduzieren des Überwachungswertes (M) durch Ändern des Kompensationssignals (i2).

7. Das Verfahren nach Anspruch 6, wobei der Überwachungswert (M) durch Ändern des Kompensationssignals (i2) auf Null gesetzt wird.

## Revendications

1. Un dispositif capteur capacitif pour mesurer au moins un paramètre d'un corps textile allongé, en particulier un fil, le dispositif capteur comprenant :
un volume de mesure (10) destiné à recevoir le corps textile,
un générateur de tension alternative (17) qui est adapté pour générer une tension alternative de mesure (Vm) avec une fréquence de base (f0)
un agencement de mesure capacitif (4) comprenant un condensateur de mesure (Cm) et un condensateur de référence (Cr) disposés en série, dans lequel le condensateur de mesure (Cm) comprend des électrodes (6b, 6c) disposées sur des côtés opposés du volume de mesure (8), et dans lequel le dispositif de mesure (4) a une sortie (OUT) disposée pour générer un signal de sortie, la sortie étant disposée entre le condensateur de mesure (Cm) et le condensateur de référence (Cr),
un amplificateur de signal (A3) pour amplifier le signal de sortie,
un circuit de compensation de décalage (34) comprenant une unité de commande (28) et un compensateur de décalage (32), dans lequel le compensateur de décalage (32) est agencé pour compenser une asymétrie entre le condensateur de mesure (Cm) et le condensateur de référence (Cr), et dans lequel l'unité de commande (28) est agencée pour commander le compensateur de décalage (32) en réponse à une amplitude du signal de sortie,
**caractérisé en ce que** le compensateur de décalage (32) comprend
un générateur de forme d'onde de compensation (S3) configuré pour générer un signal de compensation de tension alternative (i2) en synchronisme avec la tension de mesure (Vm),
dans lequel le générateur de forme d'onde de compensation (S3) est connecté à un point de correction (P1) situé le long d'un chemin de signal allant de la sortie (OUT) de l'agencement de mesure (4) à une entrée de l'amplificateur de signal (A3).

2. Le dispositif capteur selon la revendication 1, comprenant en outre un condensateur de couplage (Cc) entre le générateur de forme d'onde de compensation (S3) et le point de correction (P1).

3. Le dispositif capteur selon l'une des revendications 2, comprenant en outre une résistance (R4) en série avec le condensateur de couplage (Cc).

4. Le dispositif capteur selon la revendication 2, dans lequel une capacité Cc du condensateur de couplage (Cc), une résistance R4 de la résistance (R4) et la fréquence fondamentale f0 satisfont Cc·R4/f0 < 0.4.

5. Le dispositif capteur selon l'une des revendications précédentes, dans lequel l'unité de commande (28) est configurée pour modifier une amplitude du signal de compensation (i2) pour compenser l'asymétrie.

6. Un procédé de compensation de décalage du dispositif capteur selon l'une des revendications précédentes, comprenant les étapes suivantes
mesurer une valeur de surveillance (M) indicative d'une amplitude d'un signal de sortie de l'amplificateur de signaux (A3), et
réduire la valeur de surveillance (M) en modifiant le signal de compensation (i2).

7. Le procédé selon la revendication 6, dans lequel la valeur de surveillance (M) est mise à zéro en modifiant le signal de compensation (i2).
